# EUROPÄISCHE PATENTANMELDUNG

(11) **EP 1 127 861 A1**
(43) Veröffentlichungstag der Anmeldung: **29.08.2001**
(21) Anmeldenummer: 01102382.7
(22) Anmeldetag: 02.02.2001
(51) Int. Cl.: C07B 37/04

(54) **Verfahren zur Herstellung substituierter Benzylverbindungen und Toluolderivate**

(30) Priorität: 22.02.2000 DE 10007939
(71) Anmelder: Clariant GmbH, 65929 Frankfurt am Main (DE)
(72) Erfinder: Haber, Steffen, Dr., 76829 Landau/Pfalz (DE); Scherer, Stefan, Dr., 64572 Büttelborn (DE); Kautz, Heinz Georg, 63633 Bierstein (DE); Meudt, Andreas, Dr., 65439 Flörsheim-Weilbach (DE); Vollmüller, Frank, Dr., 60323 Frankfurt am Main (DE)

(57) **Zusammenfassung**

Das hier beschriebene erfindungsgemäße Zweistufen-Verfahren ermöglicht durch suzukiartige Kupplungsreaktionen eines Aromaten mit einer Organoborverbindung, gefolgt von einer Reduktion, einen einfachen, hohe Ausbeuten liefernden Zugang zu substituierten Benzylverbindungen und Toluolderivaten. Insbesondere eignet sich das Verfahren zur Herstellung ortho-substituierter Benzylverbindungen und Toluolderivate. Das Verfahren ist sowohl auf inter-, als auch auf intramolekulare Kupplungsreaktionen anwendbar. Als Katalysator für die Kupplungsreaktion werden Palladiumverbindungen und/oder Nickelverbindungen eingesetzt. Vorteilhafterweise werden dabei nur sehr niedrige Katalysatormengen benötigt.

## Beschreibung

Die Herstellung komplex substituierter Benzylverbindungen und Toluolderivate ist durch ältere, etablierte Verfahren, beispielsweise Gomberg-Bachmann- oder Ullmann-Kupplungen, oft nur unter Akzeptieren niedriger Ausbeuten bzw. aufwendiger Reinigungsprozesse möglich. Dies gilt insbesondere für unsymmetrisch substituierte Aromaten.
In den letzten zwanzig Jahren wurden zahlreiche Synthesemethoden entwickelt, die auf der übergangsmetallkatalysierten Kupplung zweier geeigneter Substrate beruhen. Ein Beispiel hierfür sind suzukiartige Kupplungen (Chem. Rev. 1995, 95, 2457; Tetrahedron Lett. 1979, 3437), in denen ein Bororganyl mit einem eine potentielle Abgangsgruppe tragenden Substrat unter Einsatz von Übergangsmetallen, in der Regel Palladium- oder Nickelverbindungen, in Gegenwart einer Base gekuppelt wird.
Als geeignete Abgangsgruppen kommen die am meisten verwendeten Halogene (I, Br, Cl, F) und Sulfonate (z.B. -OSO₂CH₃, -OSO₂CF₃, -OSO₂C₄F₉) in Frage, sowie eine Vielzahl weiterer, weniger oft verwendeter Gruppen (z.B. Phosphonate, Fluorsulfonate, Diazoniumsalze).

Die Kupplung von Halogentoluolen mit Bororganylen verläuft unter den in der Literatur beschriebenen Standardreaktionsbedingungen oft nur mit geringen Ausbeuten, insbesondere bei den wirtschaftlich bedeutenden Chloraromaten. Durch Einsatz von maßgeschneiderten Basen, Lösemittel- und Katalysatorsystemen ist die Reaktion in einzelnen Fällen in befriedigenden Ausbeuten durchführbar (J. Org. Chem. 1999, 64, 3804; J. Am. Chem. Soc. 1998, 120, 9722; Angew. Chem. 1998, 110(4), 492; DE-A-4326169).

Als besonders problematisch erweist sich die Kupplung sterisch gehinderter Toluole, bei denen die Abgangsgruppe in ortho-Stellung zur Methylgruppe steht. In diesen Fällen treten, besonders bei Chlor als Abgangsgruppe, vermehrt Konkurrenzreaktionen zur C/C- bzw. C/N-Verknüpfung in den Vordergrund, da die gewünschte Kupplungsreaktion im Ablauf stark gehemmt ist.
Durch Verwendung maßgeschneiderter Katalysatorsysteme läßt sich auch hier die Ausbeute verbessern. Die für die Katalyse verwendeten Liganden sind jedoch meist nur über mehrere Syntheseschritte zugänglich und damit teuer. Außerdem lassen sie sich nicht problemlos lagern oder erfordern hohe Edelmetallmengen (Angew. Chem. Int. Ed. Engl. 1999, 27(24), 3387; J. Org. Chem. 1999, 64, 10; J. Am. Chem. Soc. 1998, 120, 7369).

Das hier beschriebene erfindungsgemäße Verfahren ermöglicht durch suzukiartige Kupplungsreaktionen eines Aromaten mit einer Organoborverbindung, gefolgt von einem Reduktionsschritt, einen einfachen, hohe Ausbeuten liefernden Zugang zu substituierten Benzylverbindungen und Toluolderivaten. Insbesondere eignet sich das Verfahren zur Herstellung ortho-substituierter Benzylverbindungen und Toluolderivate. Das Verfahren ist sowohl auf intermolekulare, als auch auf intramolekulare Kupplungsreaktionen anwendbar. Vorteilhafterweise werden dabei nur sehr niedrige Katalysatormengen benötigt.

Gegenstand der Erfindung ist ein Verfahren zur Herstellung von Verbindungen der allgemeinen Formel (IV), 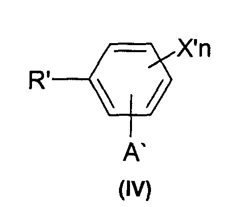 das dadurch gekennzeichnet ist, dass in einem Verfahrensschritt 1 ein Aromat der allgemeinen Formel (II), der eine Gruppe A und eine hierzu in ortho-, meta- oder para-Stellung befindliche Abgangsgruppe LG trägt, mit einer Organoborverbindung der allgemeinen Formel (I) in Gegenwart eines Palladium- und/oder Nickelkatalysators und einer Base zu einer Verbindung der Formel (III) gekuppelt wird 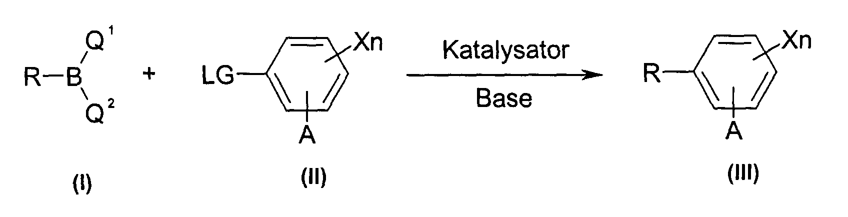 worin
- R: ein unsubstituierter oder substituierter Aryl- oder Heteroarylrest, ein unverzweigter oder verzweigter (C₁-C₁₈)-Alkylrest, ein unsubstituierter oder substituierter (C₂-C₈)-Alkenylrest oder ein unsubstituierter oder substituierter (C₂-C₈)-Alkinylrest ist, wobei Arylrest vorzugsweise einen (C₆-C₁₄)-Arylrest, insbesondere einen Phenyl-, Biphenyl- oder Naphthylrest, bedeutet und Heteroarylrest vorzugsweise einen 5- bis 7- gliedrigen Heteroarylrest mit 1 bis 3 N, S und/oder O-Atomen, wie z.B. einen Pyridin-, Pyrimidin, Pyrazin-, Pyridazin-, 1,3-Thiazol-, 1,3,4-Thiadiazol- oder einen Thiophenrest, bedeutet und wobei die Substituenten der substituierten Aryl- oder Heteroarylreste bevorzugt Halogen, CN, OH, (C₁-C₄)-Alkyl, (C₁-C₄)-Alkoxy, NO₂, CF₃, CHO, NH₂, NHAlkyl-(C₁-C₈), NAlkyl₂-(C₁-C₈), COOH, COO-Alkyl-(C₁-C₈) oder Aryl, insbesondere Phenyl, sind,
bevorzugt ein unsubstituierter oder substituierter (C₆-C₁₄)-Arylrest, ein unverzweigter oder verzweigter (C₁-C₈)- Alkylrest, ein unsubstituierter oder substituierter (C₂-C₄)-Alkenylrest oder ein unsubstituierter oder substituierter (C₂-C₄)-Alkinylrest ist und
insbesondere ein unsubstituierter oder substituierter Phenyl- oder Biphenylrest ist,
- Q¹, Q²: gleich oder verschieden sind und OH, (C₁-C₄)-Alkoxy, (C₁-C₄)-Alkyl, Phenyl oder Halogen bedeuten, zusammen eine (C₁-C₄)-Alkylendioxy-Gruppe bilden, die durch 1 bis 4 (C₁-C₄)-Alkylgruppen substituiert sein kann, zusammen eine 1,2-Phenylendioxygruppe bilden oder zusammen mit dem Boratom Teil eines Boroxinringes der Formel (V) sind, wobei die Reste R unabhängig voneinander gleich oder verschieden sind 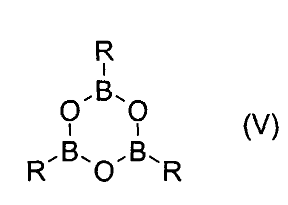
- Q¹, Q²: bevorzugt OH oder (C₁-C₄)-Alkoxy bedeuten, zusammen eine (C₁-C₃)-Alkylendioxy-Gruppe, die durch 1 bis 4 Methylgruppen substituiert sein kann, bilden oder zusammen mit dem Boratom Teil eines Boroxinringes der Formel (V) sind, besonders bevorzugt OH, Butyl- oder Isobutyloxy bedeuten, zusammen eine Ethylendioxy-, 1,1,2,2-Tetramethylethylendioxy-, Propylen-1,3-dioxy- oder Neopentyldioxy-Gruppe bilden oder zusammen mit dem Boratom Teil eines Boroxinringes der Formel (V) sind,
- A: eine Cyanogruppe oder eine Carbonylfunktion der allgemeinen Formel COR¹ bedeutet, wobei R¹ für H, einen unverzweigten oder verzweigten (C₁-C₈)-Alkylrest oder einen unsubstituierten oder substituierten Aryl- oder Heteroarylrest steht,
bevorzugt eine Cyanogruppe oder eine Carbonylfunktion der allgemeinen Formel COR¹ bedeutet, wobei R¹ für H oder einen unverzweigten oder verzweigten (C₁-C₄)-Alkylrest steht, und insbesondere eine Cyanogruppe, eine Formylgruppe oder -COCH₃ bedeutet,
- LG: eine Abgangsgruppe, wie z.B. ein Halogenatom, eine Sulfonatgruppe oder eine Diazoniumgruppe, bedeutet,
bevorzugt lod, Brom, Chlor, -OSO₂CH₃ oder -OSO₂CF₃ bedeutet,
besonders bevorzugt lod, Brom oder Chlor bedeutet
und insbesondere Chlor bedeutet und
- X: für Wasserstoff oder Substituenten aus der Gruppe Aryl, gegebenenfalls substituiert mit Substituenten vorzugsweise aus der Gruppe Halogen, CN, OH, (C₁-C₄)-Alkyl, (C₁-C₈)-Alkoxy, NO₂, CF₃, CHO, NH₂, NHAlkyl-(C₁-C₈), NAlkyl₂-(C₁-C₈), COOH, COO-Alkyl-(C₁-C₈) und Aryl; Alkyl-(C₁-C₈), gegebenenfalls verzweigt, Alkenyl-(C₁-C₈), gegebenenfalls verzweigt, Alkinyl-(C₁-C₈), gegebenenfalls verzweigt, Alkoxy-(C₁-C₈), Acyloxy-(C₁-C₈), OPhenyl, Fluor, Chlor, NO₂, NH₂, NHAlkyl-(C₁-C₈), NAlkyl₂-(C₁-C₈), OH, CN, CHO, COOH, SO₃H, SO₃-Alkyl-(C₁-C₈), SO₂NH₂, SO₂N(Alkyl-(C₁-C₈))₂, SO₂-Alkyl-(C₁-C₈), COO-Alkyl-(C₁-C₈), CONH₂, CO-Alkyl-(C₁-C₈), NHCHO, CF₃, 5-Ring-heteroaryl oder 6-Ring-heteroaryl steht, oder jeweils zwei der Substituenten X zusammen einen aliphatischen oder aromatischen 5 bis 6-gliedrigen Kohlenstoff- oder Heteroring, enthaltend C-, N-, S- und/oder O-Atome, bilden, wobei n gleich 1,2,3 oder 4 sein kann und
X bevorzugt für Wasserstoff oder Substituenten aus der Gruppe Alkyl-(C₁-C₄), Alkoxy-(C₁-C₄), Fluor, Chlor, NO₂, NH₂, NHAlkyl-(C₁-C₈), NAlkyl₂-(C₁-C₈), OH, CN, CHO und/oder COOH steht und wobei n gleich 1, 2, 3 oder 4 sein kann
und in einem Verfahrensschritt 2 die Verbindung der allgemeinen Formel (III) zur Verbindung der allgemeinen Formel (IV) reduziert wird 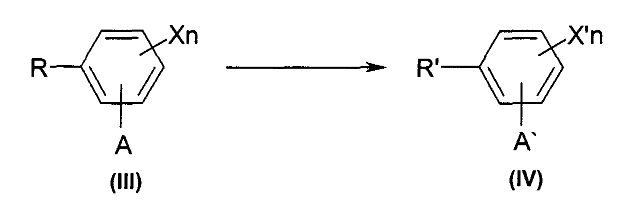 worin
- A': CH₃, CH₂NH₂,CH₂OH, CH(OH)R¹ oder CH₂ R¹ bedeutet,
- X'n: die Bedeutung von Xn oder hydriertem Xn hat und
- R': die Bedeutung von R oder hydriertem R hat.

Für die Kupplungsreaktion insbesonders bevorzugte Organoborverbindungen sind Verbindungen der allgemeinen Formel (V), wobei die Reste R unabhängig voneinander gleich oder verschieden sein können, und Verbindungen der allgemeinen Formeln (VI), (VII) oder (Vlla), 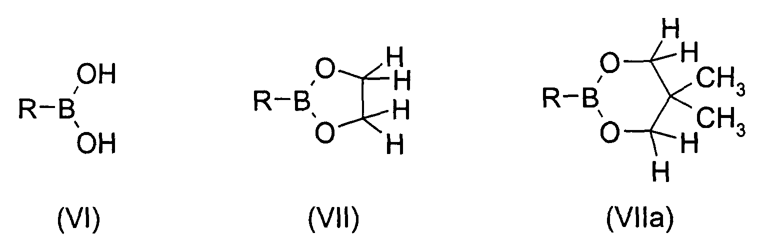 wobei auch Gemische der Verbindungen eingesetzt werden können.

Wenn die Organoborverbindung (I) Bestandteil des die Abgangsgruppe LG tragenden Aromaten (II) ist, dann verläuft die Kupplung im Sinne einer intramolekularen Reaktion.

Als Katalysatoren für den Verfahrensschritt 1 werden Palladiummetall, Palladiumverbindungen und/oder Nickelverbindungen eingesetzt. Die Katalysatoren können auch auf einem festen Träger, wie Aktivkohle oder Aluminiumoxid, aufgebracht sein.
Bevorzugt sind Palladiumkatalysatoren, in denen das Palladium in der Oxidationsstufe (0) oder (II) vorliegt, wie z.B. Palladiumketonate, Palladiumacetylacetonate, Nitrilpalladiumhalogenide, Palladiumhalogenide, Allylpalladiumhalogenide und/oder Palladiumbiscarboxylate.
Besonders bevorzugt sind Palladiumbisacetylacetonat, Bis(benzonitril)palladiumdichlorid, PdCl₂, Na₂PdCl₄, Na₂PdCl₆, Bis(acetonitril)palladiumdichlorid, Palladium-II-acetat, Bis(triphenylphosphin)palladiumdichlorid, Tetrakis(triphenylphosphin)palladium, Bis(diphenylphosphino)ferrocenpalladiumdichlorid und/oder Tetrachlorpalladiumsäure.
Die Palladiumverbindung kann auch in situ erzeugt werden, wie beispielsweise Palladium(II)acetat aus Palladium(II)chlorid und NaOAc.

Die Menge an Katalysator beträgt, bezogen auf den die Abgangsgruppe LG tragenden Aromaten (II), bevorzugt 0,001 bis 0,5 Mol-% und besonders bevorzugt 0,01 bis 0,2 Mol-%.

Der Katalysator kann phosphorhaltige Liganden enthalten oder es können dem Reaktionsgemisch phosphorhaltige Liganden separat zugesetzt werden.

Als phosphorhaltige Liganden eignen sich bevorzugt Tri-n-alkylphosphane, Triarylphosphane, Dialkylarylphosphane, Alkyldiarylphosphane und/oder Heteroarylphosphane, wie Tripyridylphosphan und Trifurylphosphan, wobei die drei Substituenten am Phosphor gleich oder verschieden sein können und wobei einer oder mehrere Substituenten die Phosphorgruppen mehrerer Phosphane verknüpfen können, wobei ein Teil dieser Verknüpfung auch ein Metallatom sein kann.

Besonders bevorzugt sind Phosphane wie Triphenylphosphan, Tri-tert-butylphosphan, Tricyclohexylphosphan, Bis(diphenylphosphino)ferrocen und/oder Tris-(3-sulfophenyl)-phosphin-Trinatriumsalz (TPPTS).

Die Gesamtkonzentration an phosphorhaltigen Liganden beträgt, bezogen auf den die Abgangsgruppe LG tragenden Aromaten (II), bevorzugt bis 1 Mol-%, besonders bevorzugt 0,001 bis 1 Mol-% und insbesonders bevorzugt 0,01 bis 0,5 Mol-%.

Die in Verfahrensschritt 1 (Kupplungsreaktion) üblicherweise verwendeten Basen sind Alkalimetallhydroxide, Erdalkalimetallhydroxyde, Alkalimetallcarbonate, Erdalkalimetallcarbonate, Alkalimetallhydrogencarbonate, Alkalimetallalkoholate, Erdalkalimetallalkoholate, Alkalimetallfluoride, primäre Amine, sekundäre Amine oder tertiäre Amine.
Bevorzugt sind Basen wie Natriumhydroxid, Kaliumhydroxid, Lithiumcarbonat, Natriumcarbonat, Kaliumcarbonat oder Kaliumfluorid.
Es können auch Gemische der Basen eingesetzt werden.
Die Menge an eingesetzter Base beträgt bevorzugt 1-10, besonders bevorzugt 1-5 und insbesonders bevorzugt 1-2.5 Moläquivalente Base bezogen auf den Aromaten (II).

Als Lösemittel für den Verfahrensschritt 1 werden Alkohole, Polyole, Polyethylenglykole, Sulfoxide oder deren Gemische verwendet.

Bevorzugte Lösemittel sind Methanol, Ethanol, Butanol, Isopropanol, Ethylenglykol, Glycerin, Tetraethylenglykol, Dimethylsulfoxid oder Gemische derselben. Besonders bevorzugt als Lösemittel sind Ethylenglykol, Methanol oder Dimethylsulfoxid. In allen Fällen können Wasser, 1,2-Dimethoxyethan, Tetrahydrofuran oder lipophile Lösemittel, wie z.B. Toluol, Xylol, Chlorbenzol oder Dichlormethan, als Cosolvens zugesetzt werden.

Zur Durchführung des Verfahrensschrittes 1 werden zweckmässigerweise die Edukte, das Lösemittel, die Base, der Katalysator und gegebenenfalls der Ligand durchmischt und bevorzugt bei einer Temperatur von 0 bis 200°C, besonders bevorzugt bei 30-170°C und insbesonders bevorzugt bei 50-150°C umgesetzt. Ausser als Eintopfreaktion kann die Reaktion auch so geführt werden, daß die verschiedenen Reaktanden im Laufe der Reaktion kontrolliert zudosiert werden, wobei unterschiedliche Dosiervarianten möglich sind.

Das molare Reaktandenverhältnis vom Aromat (II) zur Organoborverbindung (I) beträgt bevorzugt 0.9 bis 1.1.

Im Verfahrensschritt 2 des erfindungsgemäßen Verfahrens wird das Produkt aus Verfahrensschritt 1 durch Reduktion je nach Wahl der Reaktionsbedingungen zu einem Benzylderivat oder einem Toluolderivat umgesetzt.

Die Reduktion von Aldehyden, Ketonen bzw. Nitrilen der allgemeinen Formel (III) (A = CHO, COR¹ bzw. CN) zu Benzylverbindungen der allgemeinen Formel (IV) (A' = CH₂OH, CHR¹OH bzw. CH₂NH₂) oder Toluolderivaten der allgemeinen Formel (IV) (A' = CH₃, CH₂R¹) kann dabei nach den dem Fachmann geläufigen Methoden erfolgen. Bei der Reduktion können die Gruppen R und Xn in die Gruppen R' bzw. X'n umgewandelt werden. Beispielsweise können Nitrogruppen zu Aminogruppen, Alkene zu Alkanen, Nitrile zu Alkylaminen und Carbonyle zu Alkoholen reduziert werden.

Die Umsetzung von Aldehyden der allgemeinen Formel (III) (A = CHO) in Benzylalkohole der allgemeinen Formel (IV) (A' = CH₂OH) kann erfolgen durch:
- Reduktionen mit Bor- oder Aluminiumhydriden, wie Natriumborhydrid, Lithiumaluminumhydrid oder Natrium-dihydrobis(2-methoxyethoxy)aluminat, in alkoholischen Lösemitteln oder Ethern, wie Tetrahydrofuran oder Diethylether.
- Katalytische Hydrierungen mit heterogenen Palladium-, Nickel- oder Platinkatalysatoren, beispielsweise Pd auf Kohle, Raney-Nickel oder Platin auf Kohle, aber auch homogenen Hydrierkatalysatoren, beispielsweise Tris(triphenylphosphin)rhodium-(I)chlorid und elementarem Wasserstoff in Lösemitteln wie Alkoholen (z.B. MeOH, EtOH), Wasser oder Estern (z.B. Butylacetat). Die Reduktionen können drucklos oder unter Druck durchgeführt werden.

Die Umsetzung von Aldehyden der allgemeinen Formel (III) (A = CHO) in Toluolderivate der allgemeinen Formel (IV) (A' = CH₃) kann erfolgen durch:
- Katalytische Hydrierungen mit heterogenen Palladium- oder Platinkatalysatoren, beispielsweise Pd auf Kohle oder Platin auf Kohle und elementarem Wasserstoff in Lösemitteln wie Alkoholen (z.B. MeOH, EtOH), Wasser oder Estern (z.B. Butylacetat), gegebenenfalls in Gegenwart von Mineralsäuren, wie beispielsweise Schwefelsäure, Phosphorsäure oder Salzsäure, oder in Essigsäure. Die Reduktionen werden bevorzugt unter Druck und bei Temperaturen zwischen 50 und 150°C durchgeführt. Bei geeigneten Substraten ist auch eine Hydrierung ohne Lösemittelzusatz möglich.
- Transferhydrierungen mit heterogenen Palladiumkatalysatoren, z.B. Palladium auf Kohle, mit Ammoniumformiat oder Ameisensäure als Wasserstoffquellen in geeigneten Lösemitteln, wobei Ameisensäure bevorzugt ist.
- Reduktionen mit Hydrazin nach Wolff-Kishner (wasserfreies Hydrazin, Natriumalkoholate) oder nach Huang-Minlon (Hydrazinhydrat, Kalium- oder Natriumhydroxid).

Gegebenenfalls ist vor der Reduktion eine Derivatisierung der Aldehyde in die entsprechenden Imine zweckmäßig.

Die Umsetzung von Ketonen der allgemeinen Formel (III) (A = COR¹) in Benzylalkohole der allgemeinen Formel (IV) (A' = CHR¹OH) kann erfolgen durch:
- Reduktionen mit Bor- oder Aluminiumhydriden, wie Natriumborhydrid, Lithiumaluminumhydrid oder Natrium-dihydrobis(2-methoxyethoxy)aluminat in alkoholischen Lösemitteln oder Ethern, wie Tetrahydrofuran oder Diethylether.
- Katalytische Hydrierungen mit heterogenen Palladium-, Nickel- oder Platinkatalysatoren, beispielsweise Palladium, Raney-Nickel oder Platin auf Kohle, aber auch homogenen Hydrierkatalysatoren, beispielsweise Tris(triphenylphosphin)rhodium-(I)chlorid und elementarem Wasserstoff, in Lösemitteln wie Alkoholen (z.B. MeOH, EtOH), Wasser oder Estern (z.B. Butylacetat). Die Reduktionen können drucklos oder unter Druck durchgeführt werden.

Die Umsetzung von Ketonen der allgemeinen Formel (III) (A = COR¹) in Toluolderivate der allgemeinen Formel (IV) (A' = CH₂R¹) kann erfolgen durch:
- Reduktionen nach Clemmensen mit Zink und Salzsäure.
- Reduktionen nach Wolff-Kishner bzw. Huang-Minlon mit Hydrazin im alkalischen Medium.
- Katalytische Hydrierungen mit heterogenen Palladium- oder Platinkatalysatoren, beispielsweise Pd auf Kohle oder Platin auf Kohle, und elementarem Wasserstoff in Lösemitteln wie Alkoholen (z.B. MeOH, EtOH), Wasser oder Estern (z.B. Butylacetat), gegebenenfalls in Gegenwart von Mineralsäuren, wie beispielsweise Schwefelsäure, Phosphorsäure oder Salzsäure, oder in Essigsäure.
   Die Reduktionen werden bevorzugt unter Druck und bei Temperaturen zwischen 50 und 150°C durchgeführt. Bei geeigneten Substraten ist auch eine Hydrierung ohne Lösemittelzusatz möglich.

Die Umsetzung von Nitrilen der allgemeinen Formel (III) (A = CN) in Benzylamine der allgemeinen Formel (IV) (A' = CH₂NH₂) kann erfolgen durch:
- Reduktionen mit Bor- oder Aluminiumhydriden, wie Natriumborhydrid, Lithiumaluminumhydrid oder Natrium-dihydrobis(2-methoxyethoxy)aluminat in alkoholischen Lösemitteln oder Ethern, wie Tetrahydrofuran oder Diethylether.
- Katalytische Hydrierungen mit heterogenen Palladium-, Nickel- oder Platinkatalysatoren, beispielsweise Pd auf Kohle, Raney-Nickel oder Platin auf Kohle, und elementarem Wasserstoff in Lösemitteln wie Alkoholen (z.B. MeOH, EtOH), Wasser oder Estern (z.B. Butylacetat) oder Essigsäure, gegebenenfalls unter sauren Bedingungen, beispielsweise durch Zusatz von Salzsäure, oder unter acylierenden Bedingungen, beispielsweise in Gegenwart von Acetanhydrid oder Ameisensäureestern. Die Reduktionen können drucklos oder unter Druck durchgeführt werden.

Die Umsetzung von Nitrilen der allgemeinen Formel (III) (A = CN) in Toluolderivate der allgemeinen Formel (IV) (A' = CH₃) kann erfolgen durch:
- Katalytische Hydrierungen mit heterogenen Palladium-, Nickel- oder Platinkatalysatoren, beispielsweise Pd auf Kohle, Raney-Nickel oder Platin auf Kohle, und elementarem Wasserstoff in Lösemitteln wie Alkoholen (z.B. MeOH, EtOH), Wasser oder Estern (z.B. Butylacetat) oder Essigsäure gegebenenfalls unter sauren Bedingungen, beispielsweise durch Zusatz von Salzsäure, oder unter acylierenden Bedingungen, beispielsweise in Gegenwart von Acetanhydrid oder Ameisensäureestern. Die Reduktionen können drucklos oder unter Druck durchgeführt werden. Dabei sind in der Regel höhere Temperaturen zwischen 80 und 150°C notwendig.
- Hydrierungen in der Gasphase bei Temperaturen oberhalb 250°C in Gegenwart geeigneter Katalysatoren, wie beispielsweise Nickel/Kupfer-Mischkatalysatoren oder Molybdänsulfid.

Zu den verschiedenen Methoden der katalytischen Hydrierung sei an dieser Stelle z.B. auf F. Zymalkowski, "Katalytische Hydrierungen im Organisch-Chemischen Laboratorium", Ferdinand Enke Verlag, Stuttgart, 1965 verwiesen.

Die Aufarbeitung der Produkte erfolgt nach bekannten, dem Fachmann geläufigen Methoden. Beispielsweise kann das Produkt durch Extraktion oder Ausfällen aus dem Reaktionsgemisch abgetrennt und anschließend je nach Produkt z.B. durch Umkristallisation, Destillation, Rektifikation, Schmelzkristallisation, Sublimation oder Chromatographie weiter aufgearbeitet werden.

### Beispiel 1

Unter Stickstoff werden 15,5 g 2-Chloracetophenon, 31,3 g 4'-n-Pentoxybiphenyl-4-boronsäure, 7,5 g Soda und eine Mischung aus 44 mg Palladium als 22%ige wäßrige Chloridlösung, 1 ml Wasser und 720 mg einer 0.6M wäßrigen TPPTS-Lösung in 120 ml Ethylenglykol und 16 ml Wasser vorgelegt und für 4 Stunden zum Sieden erhitzt. Nach Abkühlen auf Raumtemperatur versetzt man mit 150 ml Wasser, rührt 20 Minuten intensiv nach und filtriert den verbleibenden Feststoff ab. Nach Kristallisation des Rückstandes aus Aceton und Trocknen bei 50°C im Vakuum erhält man 32 g (89%) 2-(4'-n-Pentoxy[1,1']biphenyl-4-yl)acetophenon vom Schmp. 86°C.

1 g 2-(4'-n-Pentoxy[1,1']biphenyl-4-yl)acetophenon werden in Gegenwart von 0,3 g 5% Palladium auf Kohle in 30 ml Eisessig bei Raumtemperatur und unter leichtem Wasserstoffüberdruck bei 115-120°C innerhalb von 4 Stunden hydriert. Nach Abkühlen auf Raumtemperatur und Zugabe von 30 ml Wasser filtriert man den ausgefallenen Feststoff ab, löst in Methanol, filtriert vom Katalysator ab, engt die Mutterlauge ein und trocknet das ausgefallene Produkt über Nacht bei 50°C im Vakuum. Man erhält 0,87 g (90%) 2-Ethyl-4"-n-pentoxy[1,1':4',1"]terphenyl vom Schmp. 62-65°C.

### Beispiel 2

Unter Stickstoff werden 15 g 4-Chlor-3-nitrobenzaldehyd, 13,2 g 4-Carboxyphenylboronsäure-anhydrid, 10 g Soda und eine Mischung aus 50 mg Palladium als 22%ige wäßrige Chloridlösung, 1 ml Wasser und 820 mg einer 0,6 M wäßrigen TPPTS-Lösung in 145 ml Ethylenglykol und 10 ml Wasser vorgelegt und für 4 Stunden zum Sieden erhitzt. Man versetzt mit 200 ml Wasser und säuert mit konz. Salzsäure auf pH 1-2 an, woraufhin das Produkt ausfällt. Nach Kristallisation aus Isopropanol und Trocknen im Vakuum erhält man 18,4 g (84%) 4'-Formyl-6'-nitrobiphenyl-4-carbonsäure als gelben Feststoff vom Schmp. 227-235°C. 2 g 4'-Formyl-6'-nitrobiphenyl-4-carbonsäure werden zusammen mit 827 mg 5% Palladium auf Kohle und einer Spatelspitze p-Toluolsulfonsäure in 100 ml Methanol vorgelegt und unter Durchleiten von Wasserstoff 6 Stunden bei Raumtemperatur hydriert. Man erhitzt zum Sieden, filtriert vom Katalysator ab, kühlt auf 0°C ab und reinigt den ausgefallenen Feststoff durch Aufkochen mit Ethanol. Man erhält 1,4 g (79%) 2'-Amino-4'-methylbiphenyl-4-carbonsäure vom Schmp. 273-278°C.

### Beispiel 3

15 g 5-Brom-2-anisaldehyd, 9,1 g o-Tolylboronsäureanhydrid, 500 mg Bis(triphenylphosphin)palladium(II)chlorid und 22,2 g Kaliumphosphat werden in 120 ml Dimethoxyethan vorgelegt und 3 Stunden am Rückfluß erhitzt. Nach Abkühlen auf Raumtemperatur versetzt man mit 200 ml Wasser und extrahiert dreimal mit je 200 ml Methylenchlorid. Nach Einengen und Chromatographie des Rückstandes an Kieselgel (Heptan:Methylenchlorid 1:1) erhält man 13 g (82%) 4-Methoxy-2'-methyl-biphenyl-3-carbaldehyd vom Schmp. 66-69°C.
4 g 4-Methoxy-2'-methyl-biphenyl-3-carbaldehyd werden zusammen mit 2,66 g 80%iger Hydrazinhydratlösung und 4 g Kaliumhydroxid in 40 ml Triethylenglykol vorgelegt und 2 Stunden unter Rückfluß erhitzt. Danach destilliert man bis zum Erreichen einer Sumpftemperatur von 195°C das Hydrazinhydrat/Wasser-Gemisch ab und erhitzt weitere 4 Stunden zum Sieden. Nach Abkühlen auf Raumtemperatur wird mit 50 ml Wasser versetzt und dreimal mit je 40 ml Diethylether extrahiert. Nach Einengen und Kugelrohrdestillation erhält man 2,8 g (75%) 2',3-Dimethyl-4-methoxybiphenyl vom Sdpkt. 129°C bei 15 Torr.

### Beispiel 4

15 g 2-Chlorbenzonitril, 14,8 g p-Toluolboronsäure und 28,9 g Soda werden mit 50 ml p-Xylol, 40 ml DMSO und 10 ml Wasser auf 120°C erhitzt. Bei 80°C gibt man eine Mischung aus 24,7 g Palladiumacetat und 0,55 ml 0,6 M wäßriger TPPTS-Lösung in 2,5 ml DMSO zu.
Nach beendeter Reaktion werden die Phasen getrennt. Die wäßrige Phase wird mit 50 ml Xylol gewaschen. Die vereinigten organischen Phasen werden mit 20 ml Wasser gewaschen und über Natriumsulfat getrocknet. Das Lösemittel wird eingedampft und der Rückstand aus n-Heptan kristallisiert. Man erhält 18,6 g (88%) 4'-Methylbiphenyl-2-carbonitril vom Schmp. 48-49°C.

50 g 4'-Methylbiphenyl-2-carbonitril werden zusammen mit 2,5 g 5% Palladium auf Kohle in 125 ml n-Butanol 10 Stunden unter Durchleiten von Wasserstoff bei 120°C hydriert. Nach Abfiltrieren des Katalysators destilliert man fraktionierend im Vakuum und erhält 38,8 g (82%) 2,4'-Dimethylbiphenyl mit einem Sdpkt. von 138-140°C bei 14 mbar.

### Beispiel 5

Unter Stickstoff werden 466 g 2-Chlor-5-nitrobenzaldehyd, 298 g Phenylboronsäureanhydrid, 186 g Soda und eine Mischung aus 1,04 g Palladium als 22%ige wäßrige Chloridlösung, 24 ml Wasser und 17.1g einer 0,6 M wäßrigen TPPTS-Lösung in 1600 ml Ethylenglykol und 200 ml Wasser vorgelegt und für 5 Stunden zum Sieden erhitzt. Nach Abkühlen auf Raumtemperatur filtriert man vom Feststoff ab und wäscht diesen mit 750 ml Wasser. Das Rohprodukt wird in 750 ml Isopropanol suspendiert, mit 10 g Aktivkohle versetzt, 10 Minuten zum Sieden erhitzt und anschließend heiß filtriert. Nach Abkühlen der Mutterlauge auf 5°C fällt das Produkt aus. Dieses wird abfiltriert, zweimal mit je 150 ml kaltem Isopropanol gewaschen und bei 45°C im Vakuum getrocknet. Man erhält 488 g (86%) 4-Nitrobiphenyl-2-carbaldehyd vom Schmp. 79,5°C.

68 g 4-Nitrobiphenyl-2-carbaldehyd und 44 g Anilin werden in 300 ml Xylol vorgelegt und 3 Stunden am Wasserabscheider unter Sieden erhitzt. Nach Abkühlen auf Raumtemperatur gibt man 4 g 5%Palladium auf Kohle zur Lösung und hydriert 4 Stunden bei 45 bar und 140°C. Nach Filtration des Katalysators wird fraktionierend destilliert. Man erhält 49,9 g (91%) 2-Methylbiphenyl-4-amin vom Sdpkt. 175-179°C bei 12 Torr.

## Patentansprüche

1. Verfahren zur Herstellung von Verbindungen der allgemeinen Formel (IV) 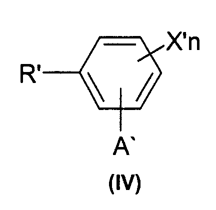 dadurch gekennzeichnet, dass in einem Verfahrensschritt 1 ein Aromat der allgemeinen Formel (II), der eine Gruppe A und eine hierzu in ortho-, meta- oder para-Stellung befindliche Abgangsgruppe LG trägt, mit einer Organoborverbindung der allgemeinen Formel (I) in Gegenwart eines Palladium- und/oder Nickelkatalysators und einer Base zu einer Verbindung der Formel (III) gekuppelt wird 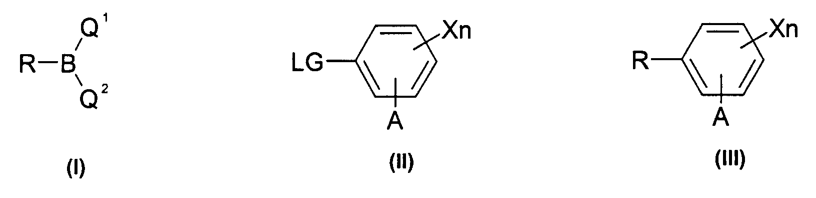 worin
R ein unsubstituierter oder substituierter Aryl- oder Heteroarylrest, ein unverzweigter oder verweigter (C₁-C₁₈)-Alkylrest, ein unsubstituierter oder substituierter (C₂-C₈)-Alkenylrest oder ein unsubstituierter oder substituierter (C₂-C₈)-Alkinylrest ist,
Q¹, Q² gleich oder verschieden sind und OH, (C₁-C₄)-Alkoxy, (C₁-C₄)-Alkyl, Phenyl oder Halogen bedeuten, zusammen eine (C₁-C₄)-Alkylendioxy-Gruppe, die gegebenenfalls durch 1 bis 4 (C₁-C₄)-Alkylgruppen substituiert sein kann, oder eine 1,2-Phenylendioxygruppe bilden oder zusammen mit dem Boratom Teil eines Boroxinringes der Formel (V) sind, wobei die Reste R unabhängig voneinander gleich oder verschieden sind, 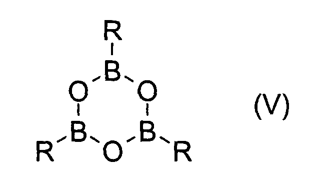
A eine Cyanogruppe oder eine Carbonylfunktion der allgemeinen Formel COR¹ bedeutet, wobei R¹ für H, einen unverzweigten oder verzweigten (C₁-C₈)-Alkylrest oder einen unsubstituierten oder substituierten Aryl- oder Heteroarylrest steht,
LG eine Abgangsgruppe bedeutet,
X für Wasserstoff oder einen Substituenten aus der Gruppe Aryl, gegebenenfalls substituiert, Alkyl-(C₁-C₈), gegebenenfalls verzweigt, Alkenyl-(C₁-C₈), gegebenenfalls verzweigt, Alkinyl-(C₁-C₈), gegebenenfalls verzweigt, Alkoxy-(C₁-C₈), Acyloxy-(C₁-C₈), OPhenyl, Fluor, Chlor, NO₂, NH₂, NHAlkyl-(C₁-C₈), NAlkyl₂-(C₁-C₈), OH, CN, CHO, COOH, SO₃H, SO₃-Alkyl-(C₁-C₈), SO₂NH₂, SO₂N(Alkyl-(C₁-C₈))2, SO₂-Alkyl-(C₁-C₈), COO-Alkyl-(C₁-C₈), CONH₂, CO-Alkyl-(C₁-C₈), NHCHO, CF₃, 5-Ring-heteroaryl oder 6-Ring-heteroaryl steht, oder jeweils zwei der Substituenten X zusammen einen aliphatischen oder aromatischen 5-6 gliedrigen Kohlenstoff- oder Heteroring, enthaltend C-, N-, S- und/oder O- Atome, bilden und wobei n gleich 1, 2, 3 oder 4 sein kann,
und in einem Verfahrensschritt 2 die Verbindung der allgemeinen Formel (III) zur Verbindung der allgemeinen Formel (IV) reduziert wird, worin
A' CH₃, CH₂NH₂,CH₂OH, CH(OH)R¹ oder CH₂ R¹ bedeutet,
X'n die Bedeutung von Xn oder hydriertem Xn hat und
R' die Bedeutung von R oder hydriertem R hat.

2. Verfahren nach Anspruch 1, dadurch gekennzeichnet, dass R für einen unsubstituierten oder substituierten (C₆-C₁₄)-Arylrest, einen unverzweigten oder verzweigten (C₁-C₈)- Alkylrest, einen unsubstituierten oder substituierten (C₂-C₄)-Alkenylrest oder einen substituierten oder unsubstituierten (C₂-C₄)-Alkinylrest steht.

3. Verfahren nach Anspruch 1 oder 2, dadurch gekennzeichnet, dass Q¹, Q² gleich oder verschieden sind und OH, Butyl- oder Isobutyloxy bedeuten, zusammen eine Ethylendioxy-, 1,1,2,2-Tetramethylethylendioxy-, Propylen-1,3-dioxy- oder Neopentyldioxy-Gruppe bilden oder zusammen mit dem Boratom Teil eines Boroxinringes der Formel (V) sind.

4. Verfahren nach mindestens einem der Ansprüche 1 bis 3, dadurch gekennzeichnet, dass A eine Cyanogruppe oder eine Carbonylfunktion der allgemeinen Formel COR¹ bedeutet, wobei R¹ für H oder einen unverzweigten oder verzweigten (C₁-C₄)-Alkylrest steht.

5. Verfahren nach mindestens einem der Ansprüche 1 bis 4, dadurch gekennzeichnet, dass LG für lod, Brom, Chlor, -OSO₂CH₃ oder -OSO₂CF₃ steht.

6. Verfahren nach mindestens einem der Ansprüche 1 bis 5, dadurch gekennzeichnet, dass X für Wasserstoff, Alkyl-(C₁-C₄), Alkoxy-(C₁-C₄), Fluor, Chlor, NO₂, NH₂, NHAlkyl-(C₁-C₈), NAlkyl₂-(C₁-C₈), OH, CN, CHO und/oder COOH steht und wobei n gleich 1, 2, 3 oder 4 sein kann.

7. Verfahren nach mindestens einem der Ansprüche 1 bis 6, dadurch gekennzeichnet, dass es sich bei den Palladiumkatalysatoren um Palladiumbisacetylacetonat, Bis(benzonitril)palladiumdichlorid, PdCl₂, Na₂PdCl₄, Na₂PdCl₆, Bis(acetonitril)palladiumdichlorid, Palladium-II-acetat, Bis(triphenylphosphin)palladiumdichlorid, Tetrakis-(triphenylphosphin)palladium, Bis(diphenylphosphino)ferrocenpalladiumdichlorid und/oder Tetrachlorpalladiumsäure handelt.

8. Verfahren nach mindestens einem der Ansprüche 1 bis 7, dadurch gekennzeichnet, dass die Menge an Katalysator aus Verfahrenschritt 1, bezogen auf den die Gruppe LG tragenden Aromaten (II), 0,001 bis 0,5 Mol-% beträgt.

9. Verfahren nach mindestens einem der Ansprüche 1 bis 8, dadurch gekennzeichnet, dass der Katalysator aus Verfahrensschritt 1 phosphorhaltige Liganden aus der Gruppe der Tri-n-alkylphosphane, Triarylphosphane, Dialkylarylphosphane, Alkyldiarylphosphane und Heteroarylphosphane enthält, wobei die drei Substituenten am Phosphor gleich oder verschieden sein können und wobei einer oder mehrere Substituenten die Phosphorgruppen mehrerer Phosphane verknüpfen können, wobei ein Teil dieser Verknüpfung auch ein Metallatom sein kann.

10. Verfahren nach Anspruch 9, dadurch gekennzeichnet, dass es sich bei den phosphorhaltigen Liganden um Triphenylphosphan, Tri-tert-butylphosphan, Tricyclohexylphosphan, Bis(diphenylphosphino)ferrocen und/oder Tris-(3-sulfophenyl)-phosphin-Trinatriumsalz handelt.

11. Verfahren nach Anspruch 9 oder 10, dadurch gekennzeichnet, dass die Gesamtkonzentration an phosphorhaltigen Liganden, bezogen auf den die Gruppe LG tragenden Aromaten (II), 0,001 bis 1 Mol-% beträgt.

12. Verfahren nach mindestens einem der Ansprüche 1 bis 11, dadurch gekennzeichnet, dass es sich bei den Basen aus Verfahrenschritt 1 um Alkalimetallhydroxide, Erdalkalimetallhydroxyde, Alkalimetallcarbonate, Erdalkalimetallcarbonate, Alkalimetallhydrogencarbonate, Alkalimetallalkoholate, Erdalkalimetallalkoholate, Alkalimetallfluoride, primäre Amine, sekundäre Amine und/oder tertiäre Amine handelt.

13. Verfahren nach mindestens einem der Ansprüche 1 bis 12, dadurch gekennzeichnet, dass Verfahrenschritt 1 in Gegenwart von Wasser und/oder eines Lösemittels aus der Gruppe Alkohole, Polyole, Polyethylenglykole, Sulfoxide und deren Gemische durchgeführt wird.

14. Verfahren nach mindestens einem der Ansprüche 1 bis 13, dadurch gekennzeichnet, dass zur Durchführung des Verfahrensschrittes 1 die Edukte, das Lösemittel, die Base, der Katalysator und gegebenenfalls der Ligand durchmischt werden und bei einer Temperatur von 0 bis 200°C umgesetzt werden.

15. Verfahren nach mindestens einem der Ansprüche 1 bis 14, dadurch gekennzeichnet, dass es sich bei Verfahrensschritt 2 um eine katalytische Hydrierung mit elementarem Wasserstoff, eine Reduktion mit Metallhydriden, eine Clemmensen-Reduktion, eine Wolff-Kishner-Reduktion, eine Reduktion nach Huang-Minlon und/oder eine Transferhydrierung handelt.
